# EUROPEAN PATENT APPLICATION

(11) **EP 3 264 082 A1**
(43) Date of publication of application: **03.01.2018**
(21) Application number: 16755913.7
(22) Date of filing: 26.02.2016
(51) Int. Cl.: G01N 33/53, G01N 33/68, C07K 14/65

(54) **USE OF IGF-1 AS PROGNOSTIC FACTOR IN CALCIFICATION OF AORTIC VALVES**

(30) Priority: 26.02.2015 KR 20150027375
(71) Applicant: The Asan Foundation, Seoul 05505 (KR); University Of Ulsan Foundation For Industry Cooperation, Ulsan 44610 (KR)
(72) Inventor: CHANG, Eun Ju, Seoul 05502 (KR); SONG, Jae Kwan, Seoul 05507 (KR)
(74) Representative: dompatent von Kreisler Selting Werner - Partnerschaft von Patent- und Rechtsanwälten mbB
(86) International application number: PCT/KR2016/001906
(87) International publication number: WO 2016/137263

(57) **Abstract**

The present invention relates to: a composition for diagnosing aortic valve calcification diseases, containing a preparation, which measures the protein level of deactivated IGF-1, wherein the composition is capable of diagnosing aortic valve calcification diseases involving calcification of aortic valves; a kit for diagnosing aortic valve calcification diseases, comprising the composition; a method for detecting deactivated IGF-1 so as to provide information necessary in the diagnosis of aortic valve calcification diseases; and a method for detecting deactivated IGF-1 so as to provide information necessary in the diagnosis of the progression level of aortic valve calcification diseases. According to the present invention, when using the composition or the kit for diagnosing aortic valve calcification diseases, it is possible to determine whether aortic valve calcification diseases, which involve the calcification of aortic valves, have occurred, and to determine the progression level of aortic valve calcification diseases which have already occurred, and thus the composition or the kit of the present invention can be used for more effectively diagnosing and treating aortic valve calcification diseases.

<110>    THE ASAN FOUNDATION
         UNIVERSITY OF ULSAN FOUNDATION FOR INDUSTRY COOPERATION

<120>    Uses of IGF-1 as prognostic factor for aortic valvular

<130>    1708

<150>    KR 10-2015-0027375
<151>    2015-02-25

<160>    2

<170>    KopatentIn 2.0

<210>    1
<211>    153
<212>    PRT
<213>    Artificial Sequence

<220>
<223>    recombinant IGF-1


<400>    1
Met Gly Lys Ile Ser Ser Leu Pro Thr Gln Leu Phe Lys Cys Cys Phe
  1               5                  10                  15

Cys Asp Phe Leu Lys Val Lys Met His Thr Met Ser Ser Ser His Leu
             20                  25                  30

Phe Tyr Leu Ala Leu Cys Leu Leu Thr Phe Thr Ser Ser Ala Thr Ala
         35                  40                  45

Gly Pro Glu Thr Leu Cys Gly Ala Glu Leu Val Asp Ala Leu Gln Phe
     50                  55                  60

Val Cys Gly Asp Arg Gly Phe Tyr Phe Asn Lys Pro Thr Gly Tyr Gly
 65                  70                  75                  80

Ser Ser Ser Arg Arg Ala Pro Gln Thr Gly Ile Val Asp Glu Cys Cys
                 85                  90                  95

Phe Arg Ser Cys Asp Leu Arg Arg Leu Glu Met Tyr Cys Ala Pro Leu
            100                 105                 110

Lys Pro Ala Lys Ser Ala Arg Ser Val Arg Ala Gln Arg His Thr Asp
        115                 120                 125

Met Pro Lys Thr Gln Lys Glu Val His Leu Lys Asn Ala Ser Arg Gly
    130                 135                 140

Ser Ala Gly Asn Lys Asn Tyr Arg Met
145                 150


<210>    2
<211>    151
<212>    PRT
<213>    Artificial Sequence

<220>
<223>    inactivated IGF-1

<400>  2
Lys Ile Ser Ser Leu Pro Thr Gln Leu Phe Lys Cys Cys Phe Cys Asp
1               5                   10                  15

Phe Leu Lys Val Lys Met His Thr Met Ser Ser Ser His Leu Phe Tyr
            20                  25                  30

Leu Ala Leu Cys Leu Leu Thr Phe Thr Ser Ser Ala Thr Ala Gly Pro
        35                  40                  45

Glu Thr Leu Cys Gly Ala Glu Leu Val Asp Ala Leu Gln Phe Val Cys
    50                  55                  60

Gly Asp Arg Gly Phe Tyr Phe Asn Lys Pro Thr Gly Tyr Gly Ser Ser
65                  70                  75                  80

Ser Arg Arg Ala Pro Gln Thr Gly Ile Val Asp Glu Cys Cys Phe Arg
                85                  90                  95

Ser Cys Asp Leu Arg Arg Leu Glu Met Tyr Cys Ala Pro Leu Lys Pro
            100                 105                 110

Ala Lys Ser Ala Arg Ser Val Arg Ala Gln Arg His Thr Asp Met Pro
        115                 120                 125

Lys Thr Gln Lys Glu Val His Leu Lys Asn Ala Ser Arg Gly Ser Ala
    130                 135                 140

Gly Asn Lys Asn Tyr Arg Met
145                 150

## Description

### [Technical Field]

The present disclosure relates to a use of IGF-1 as a prognostic factor in calcification of aortic valves, and more specifically, to a composition for diagnosing an aortic valve calcification disease, capable of diagnosing an aortic valve calcification disease involving calcification of aortic valves, comprising an agent for measuring a protein level of inactivated IGF-1, a kit for diagnosing an aortic valve calcification disease comprising the composition, a method for detecting inactivated IGF-1 to provide information necessary in the diagnosis of an aortic valve calcification disease, and a method for detecting inactivated IGF-1 to provide information necessary in the diagnosis of a progression level of the aortic valve calcification disease.

### [Background Art]

The heart has four valves that guide the flow of blood forward. Two of these are located in the left heart through which oxygenated blood flows, and the other two are located in the right heart through which deoxygenated blood flows. The valve on the left side is the mitral valve and the aortic valve, and the valve on the right side is the tricuspid valve and the pulmonary valve. Among them, the aortic valve is located between the left ventricle and the aorta, and is designed to withstand the highest pressure. The aortic valve is normally comprised of three valve leaflets, but sometimes may have two or four valve leaflets. Each valve leaflet is attached to the aortic wall by the aortic annulus, and there is a space called Sinus of Valsalva, between the aortic wall and the valve leaflet. In the normal case where the aortic valve is composed of three valve leaflets, each valve leaf is referred to as a left coronary artery leaflet, a right coronary artery leaflet, and a non-coronary artery leaflet. The area where the valve leaf meets the valve leaf is called a commissure. Thus, in the normal case where there are three valves leaflets, three commissures are present near the aortic wall, and the center of each valve leaflet is called valve cusp. Each valve leaflet is not planar, but has a concave shape when viewing from the Sinus of Valsalva, which is similar to a rose petal shape. All of the aortic valve, the aortic annulus, and the Sinus of Valsalva between the aortic wall and the valve leaflet are called an aortic root. Therefore, the aortic root is a trapezoidal cylinder, wherein an aortic annulus located in a lower part is bounded by a sinotubular junction located in an upper part, and the aortic valve leaflet is located therebetween. Accordingly, the aortic valve begins to open by twisting and tilting of the aortic root, is completely open while blood flows forward by contraction of a left ventricle, and creates a closure phase in which the valve leaflets are occluded by aortic pressure when blood flowing forward is reduced by relaxation of the left ventricle.

In order to complete the closure of the aortic valves, a diameter of the sinotubular junction should be maintained so that the valve leaflets are able to lean against each other, wherein when the operation is abnormal, an aortic valve calcification disease may be induced. For example, the aortic valve calcification disease may be divided into two types, a single lesion in the leaflet itself and a lesion in the aortic root, wherein a mixed lesion may exist between extreme ends. In the early stages of aortic valve calcification disease, only the lesion of the valve leaflet itself may be present, but in most cases, as time elapses, structural deformation of the peripheral aortic root may also be caused, and conversely, the lesion of the aortic root may prevent occlusion of the valve leaflets. As a method for treating such aortic valve calcification disease, a method for constructing a prosthetic valve and inserting it through a surgical operation is used, and various studies are being conducted to improve a success rate of such a procedure. For example, Korean Patent No. 100370 discloses sinkhole bileaflet polymer heart valve (abbreviated as a sinkhole leaflet) excellent in blood compatibility and durability and usable as heart valves such as a temporary artificial valve for blood pump, a total artificial heart (TAH) for replacement, and a ventricular assist device (VAD), etc. Korean Patent No. 1258213 discloses that a pericardial artificial heart valve conduit and a manufacturing method thereof, using a bovine pericardium or a porcine pericardium that performs function of a prosthetic valve to prevent the backflow of blood, and a preparation method thereof, wherein the bovine pericardium and the porcine pericardium are wrapped around a mold so that the bovine pericardium or the porcine pericardium is formed into a tube shape to be supported on the inner wall of the pulmonary artery.

Meanwhile, in order to diagnose the aortic valve calcification disease, it is necessary to anatomically check the condition of the valve, but it is practically impossible to check the condition of the valve anatomically. Therefore, a method for indirectly diagnosing whether the aortic valve calcification disease occurs by combining cardiovascular diagnosis data of a patient is used, but a diagnostic success rate is very low. Further, since the therapeutic prognosis after the treatment for the aortic valve calcification disease is also confirmed by the same method, there is a need for a method for confirming whether the aortic valve calcification disease occurs or the progression level thereof, but so far, no method has yet been developed yet.

Under these circumstances, the present inventors have made extensive efforts to develop a method for diagnosing whether the aortic valve calcification disease occurs, found that when valve calcification, which is one of the symptoms of the aortic valve calcification disease, progressed, a level of inactivated IGF-1 was increased simultaneously, and thus, the onset of the aortic valve calcification disease could be diagnosed by measuring a level change of inactivated IGF-1, and completed the present disclosure.

### [Summary of Invention]

An object of the present disclosure is to provide a composition for diagnosing an aortic valve calcification disease comprising an agent for measuring a protein level of inactivated insulin-like growth factor type 1 (IGF-1).

Another object of the present disclosure is to provide a kit for diagnosing an aortic valve calcification disease comprising the composition.

Still another object of the present disclosure is to provide a method for detecting inactivated IGF-1 to provide information necessary in the diagnosis of an aortic valve calcification disease.

Still another object of the present disclosure is to provide a method for detecting inactivated IGF-1 to provide information necessary in the diagnosis of a progression level of an aortic valve calcification disease.

The present inventors conducted various studies to develop a method for diagnosing whether the aortic valve calcification disease, which is a type of aortic valve disease (AVD), occurs or a progression level thereof, and focused on inactivated IGF-1 produced by DPP-4. The present inventor found that the DPP-4 or the inactivated IGF-1 produced by the DPP-4 was increased in the patient in which the aortic valve calcification disease was induced rather than in normal people, increased in the patient in which the calcification progressed rather than the patient in which the calcification did not progress among the patients in which the aortic valve calcification disease was induced, and further increased in the patient with high calcification severity rather than the patient with low calcification severity even among the patients in which the aortic valve calcification disease with the progress of calcification was induced.

Accordingly, it was confirmed that whether the aortic valve calcification disease was induced or the progression state thereof could be diagnosed by measuring an expression level of inactivated IGF-1 in the plasma of a patient suspected of having the onset of the aortic valve calcification disease or a patient in which the onset of the aortic valve calcification disease was confirmed. As described above, a method for integrally diagnosing whether the aortic valve calcification disease occurs or the progression state thereof by measuring the expression level of inactivated IGF-1, has not been known so far.

In order to achieve the foregoing objects, the present disclosure provides a composition for diagnosing an aortic valve calcification disease, comprising an agent for measuring a protein level of inactivated IGF-1. Further, the present disclosure provides a method for providing information for diagnosing an aortic valve calcification disease comprising measuring a protein level of inactivated IGF-1. Further, the present disclosure provides a method for diagnosing an aortic valve calcification disease comprising measuring a protein level of inactivated IGF-1.

### [Description of Drawings]

FIG. 1 is a schematic diagram showing an mechanism of action on DPP-4 and IGF-1 involved in an onset of an aortic valve calcification disease.
FIG. 2a are image and graph showing verification of the effect of DPP-4 on an action of IGF-1 inhibiting osteocytogenesis in aortic valve vascular smooth muscle tissue of wild-type mice or eNOS-deficient mice.
FIG. 2b is a Western blot analysis image showing a change in phosphorylation level of ERK or Akt which is a protein involved in intracellular signaling according to whether the DPP-4 is treated in the eNOS-deficient mice treated with IGF-1.
FIG. 3a is a graph showing comparison results of concentrations of DPP-4 protein contained in the plasma of wild-type mice or eNOS-deficient mice.
FIG. 3b is a graph showing comparison results of protein concentrations of inactivated IGF-1 bound to IGFBP3 contained in the plasma of wild-type mice or eNOS-deficient mice treated or not treated with sitagliptin.
FIG. 4a is a graph showing comparison results of concentrations of DPP-4 protein contained in the plasma of normal people or a patient in which the aortic valve calcification disease is induced.
FIG. 4b is a graph showing comparison results of concentrations of DPP-4 protein contained in the plasma of a patient in which the calcification of the aortic valve does not progress or the calcification progresses among the patients in which the aortic valve calcification disease is induced.
FIG. 4c is a graph showing comparison results of concentrations of activated DPP-4 protein contained in the plasma of a patient in which the calcification of the aortic valve does not progress or a patient in which the calcification progresses among the patients in which the aortic valve calcification disease is induced.
FIG. 4d is a graph showing comparison results of concentrations of DPP-4 protein contained in the plasma of a patient in which the aortic valve calcification disease with severity 1 or 2 is induced.
FIG. 4e is a graph showing comparison results of concentrations of inactivated IGF-1 protein contained in the plasma of a patient in which the calcification of the aortic valve does not progress or a patient in which the calcification progresses among the patients in which the aortic valve calcification disease is induced.
FIG. 4f is a graph showing comparison results of concentrations of inactivated IGF-1 protein contained in the plasma of a patient in which the aortic valve calcification disease without the calcification severity is induced or a patient in which the aortic valve calcification disease with the calcification severity is induced, among the patients in which the aortic valve calcification disease with the progress of calcification is induced.

### [Best Mode]

As far as it is not defined in other ways, all technical and scientific terms used in the present specification have the same meaning as being generally appreciated by those skilled in the art to which the present disclosure pertains. In general, the nomenclature used in the present specification is well known in technical fields and generally used.

A term "insulin-like growth factor type 1 (IGF-1) of the present disclosure refers to a polypeptide having a size of about 7.5 kDa present in a plasma of mammals, is detected in most tissues, and is known to promote differentiation and proliferation of a wide variety of mammalian cells. It is known that the IGF-1 binds to an IGF-1 receptor (insulin-like growth factor 1 receptor: IGF-1R) located on a cell surface to regulate cell function and play an important role in early differentiation, but it does not play a particularly important role in normal adults. Specific base sequence information of gene encoding the IGF-1 or amino acid sequence information of protein encoding the IGF-1 is known in NCBI (GenBank: NM_000618, NP_000609, etc.).

In the present disclosure, the IGF-1 may include all peptides comprising an amino acid sequence of SEQ ID NO: 1, or having various amino acid sequences added to the N-terminal or C-terminal of the amino acid sequence of SEQ ID NO: 1. Further, the IGF-1 may additionally comprise an amino acid sequence designed for a specific purpose to increase stability of the targeting sequence, tag, labeled residue, half-life or peptide.

In addition, variant proteins in which part of amino acids of the amino acid sequence of SEQ ID NO: 1 are mutated by addition, substitution, deletion, or the like, may be included in the category of the IGF-1 provided in the present disclosure.

In particular, the IGF-1 provided in the present disclosure may include a polypeptide having an amino acid sequence of SEQ ID NO: 1 and a sequence having one or more different amino acid residues. Amino acid exchanges in proteins and polypeptides that do not entirely change a molecular activity are known in the art. The amino acid exchanges that most commonly occur include exchanges between amino acid residues such as Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. Further, the IGF-1 may include a protein having increased structural stability or increased protein activity against heat, pH, etc., of the protein due to mutation or modification of the amino acid sequence.

For example, the IGF-1 provided by the present disclosure may comprise an amino acid sequence having 80% or more homology to the amino acid sequence of SEQ ID NO: 1. As another example, the IGF-1 may be composed of an amino acid sequence having 90% or more homology to the amino acid sequence of SEQ ID NO: 1, and as still another example, the IGF-1 may comprise an amino acid sequence having 95% or more homology to the amino acid sequence of SEQ ID NO: 1.

In the present disclosure, the IGF-1 may be interpreted as a material that exhibits an effect of alleviating the calcification of the aortic valve induced by the aortic valve calcification disease.

A term "inactivated IGF-1" of the present disclosure refers inactivated IGF-1 in which a function of the IGF-1 is not shown due to cleavage of a part of an N-terminal amino acid of the IGF-1.

In the present disclosure, the inactivated IGF-1 is present in a state in which an N-terminal amino acid thereof is cleaved by DPP-4 in patients having a possibility of inducing the aortic valve calcification disease or patients who already have the aortic valve calcification disease, and thus, the inactivated IGF-1 may be interpreted as a biomarker that is usable to diagnose whether the aortic valve calcification disease occurs or the progression level thereof. As an example, the inactivated IGF-1 may comprise an amino acid sequence in which 2 to 10 amino acids at the N-terminal of IGF-1 are cleaved; as another example, may comprise an amino acid sequence in which 2 to 5 amino acids at the N-terminal of IGF-1 are cleaved; and as another example, may comprise an amino acid sequence in which 2 amino acids at the N-terminal of IGF-1 are cleaved. For example, in the present disclosure, two amino acids at the N-terminal of IGF-1 cleaved by DPP-4, which is represented by the amino acid sequence of SEQ ID NO: 2, are used.

Meanwhile, the agent for measuring a protein level of the inactivated IGF-1 may be an agent for detecting the inactivated IGF-1 itself. For example, the agent may be a protein capable of specifically binding to the inactivated IGF-1, i.e., IGF-binding protein 3 (IGFBP3). For example, in the present disclosure, a level of inactivated IGF-1 contained in a sample is measured by measuring a level of total IGF-1 contained in a sample of a patient, reacting the sample with an IGFBP3-coated ELISA kit, followed by washing to select only the inactivated IGF-1 contained in the sample, and adding a labeled anti-IGF-1 antibody to measure the level of inactivated IGF-1.

A term "DPP-4 (dipeptidyl-peptidase 4)" of the present disclosure refers to an antigenic enzyme having characteristics of a membrane-bound glycoprotein expressed on most cell surfaces, also referred to as CD26, and is generally known to be involved in immune regulation, signal transduction, and apoptosis processes. The DPP-4 exhibits an activity of cleaving two peptides including proline which is structurally present at the N-terminal. Specific base sequence information of gene encoding the DDP-4 or amino acid sequence information of protein encoding the DDP-4 is known in NCBI (GenBank: NM_001935, NP_001926, etc.).

Term "aortic valve calcification disease" of the present disclosure refers to a disease caused when inorganic ions such as calcium, etc., are deposited on the aortic valve due to various reasons, and the aortic valve is calcified, resulting in aortic valve stenosis, which may cause abnormalities in the function of regulating the blood flow through the aorta.

A term "diagnosis" of the present disclosure means confirmation of the presence or the characteristic of pathological conditions. In the present disclosure, the diagnosis may be interpreted as all behaviors to confirm whether the aortic valve calcification disease involving the calcification of the valve occurs or the progression level thereof.

A phrase "the agent for measuring a protein level" refers an agent used in a method for measuring a level of a target protein contained in a sample, and preferably may be an antibody or an aptamer used in methods such as western blotting, enzyme linked immunosorbent assay (ELISA), radioimmunoassay (RIA), radioimmunodiffusion, ouchterlony immunodiffusion, rocket immunoelectrophoresis, tissue immunostaining, immunoprecipitation assay, complement fixation assay, FACS, protein chip assay, etc.

A term "antibody" of the present disclosure means a proteinaceous molecule capable of specifically binding to an antigenic site of a protein or a peptide molecule, wherein the antibody may be prepared by cloning each gene into an expression vector according to conventional methods to obtain a protein encoded by the marker gene, and preparing the obtained protein by a conventional method. The antibody is not particularly limited in view of a form, and may include a polyclonal antibody, a monoclonal antibody, or a portion of an antibody having an antigen binding property, and not only all immunoglobulin antibodies but also special antibodies such as humanized antibody, etc. Further, the antibody includes a functional fragment of an antibody molecule as well as a complete form including two light chains having the entire length and two heavy chains having the entire length. The functional fragment of the antibody molecule means a fragment possessing an antigen binding function at least, and may include Fab, F(ab'), F(ab')2, Fv, etc.

In the present disclosure, the antibody may be an antibody capable of specifically binding to the inactivated IGF-1 protein, preferably, a polyclonal antibody, a monoclonal antibody, or a portion thereof capable of specifically binding to the inactivated IGF-1 protein.

A term "aptamer" of the present disclosure refers to a material capable of specifically binding to a target material to be detected in a sample, and is a single-stranded nucleic acid (DNA, RNA or modified nucleic acid) having a stable tertiary structure by itself. The presence of the target material in the sample may be specifically confirmed through the binding. The preparation of the aptamer may be performed by determining and synthesizing an oligonucleotide having a selective and high binding ability to a target protein to be identified according to a general method of preparing an aptamer, and then modifying the 5' end or 3' end of the oligonucleotide to -SH, -COOH, -OH or -NH₂ so that it is able to be bound to a linker's functional group.

In the present disclosure, the aptamer may be an aptamer capable of specifically binding to the inactivated IGF-1 protein, preferably, a DNA aptamer capable of specifically binding to the inactivated IGF-1 protein.

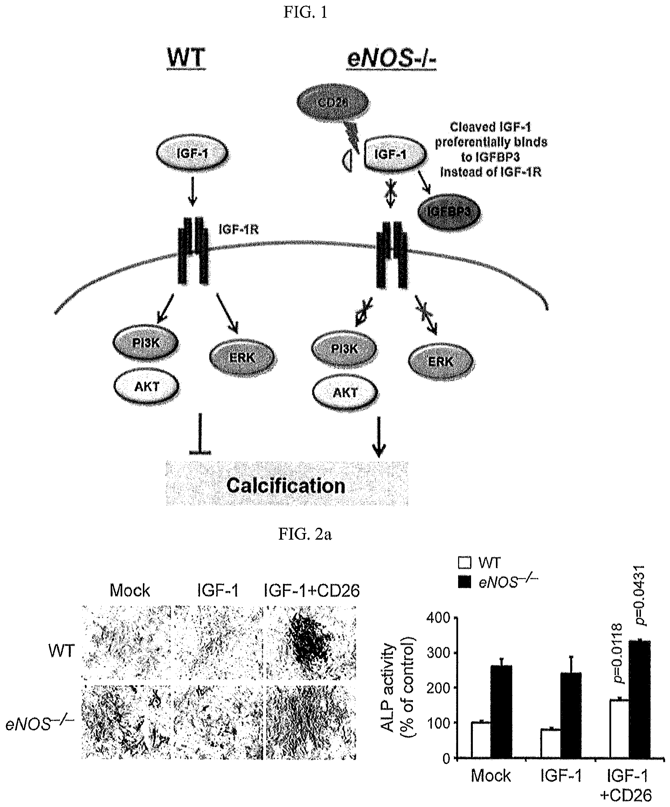

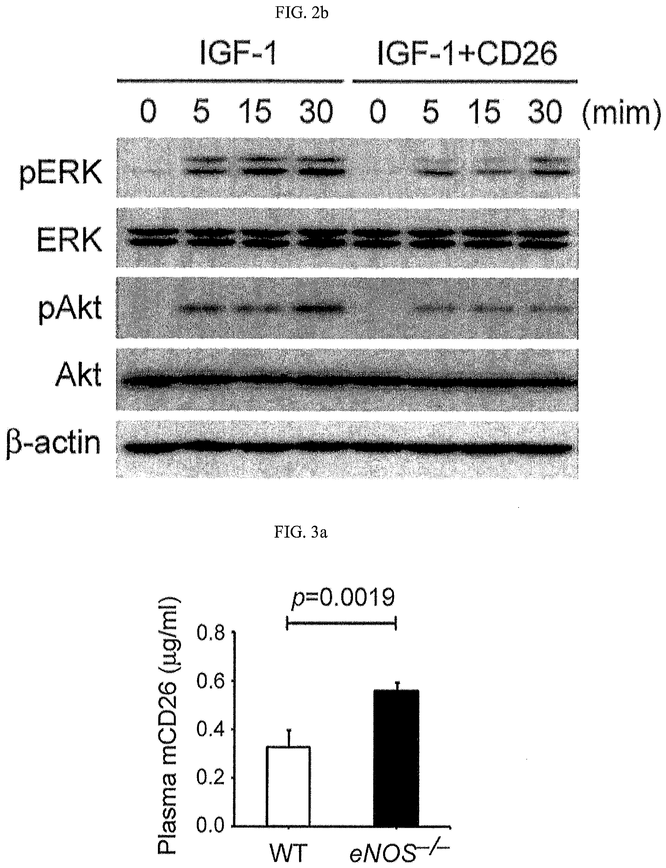

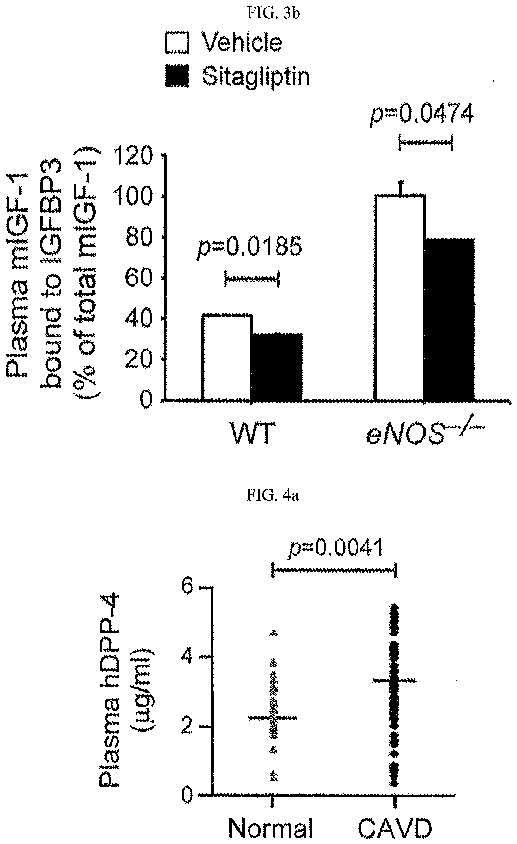

According to an exemplary embodiment of the present disclosure, as a result of analyzing effects of IGF-1 and DPP-4 on osteocytogenesis of the aortic valve vascular smooth muscle tissue of the animal model with the aortic valve calcification, it was confirmed that the osteocytogenesis of the tissue tended to be alleviated by the treatment of IGF-1, but the osteocytogenesis of the tissue was intensified by the addition of CD26 (FIG. 2a), the protein involved in intracellular signaling associated with IGF-1 was inactivated by DPP-4 (FIG. 2b), the plasma of model mice with the aortic valve calcification, rather than the plasma of the wild-type mice, contained a high concentration of DPP-4 protein (FIG. 3a), and a protein concentration of inactivated IGF-1 bound to IGFBP3 was reduced by treatment with sitagliptin which is an inhibitor of DPP-4 (FIG. 3b).

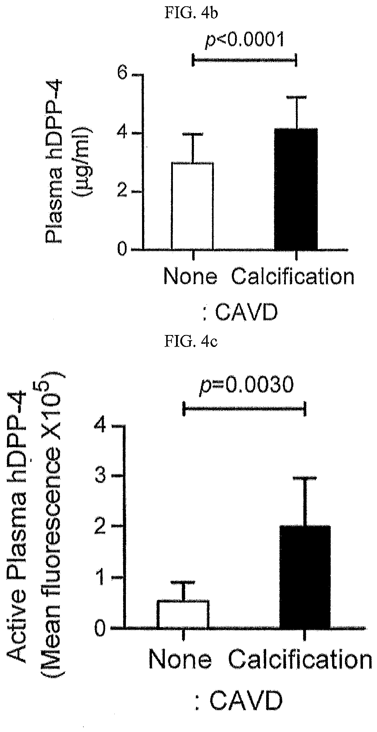

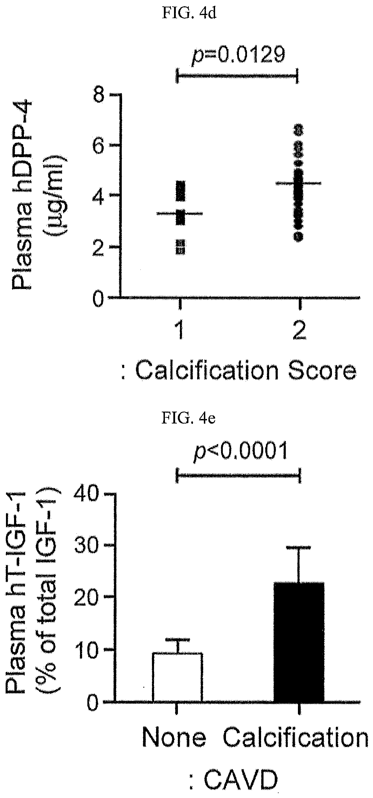

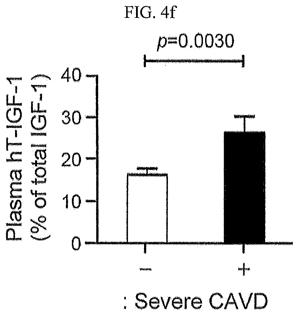

Meanwhile, as a result of verifying the above results using patients in which the aortic valve calcification disease are induced, it was confirmed that the plasma of patients, rather than the plasma of normal people, contained a high concentration of DPP-4 protein (FIG. 4a), the plasma of the patient in which the calcification of the aortic valve progressed, rather than the plasma of the patient in which the calcification of the aortic valve did not progress among the patients, contained a high concentration of DPP-4 protein (FIG. 4b), the plasma of the patient in which the calcification of the aortic valve progressed, rather than the plasma of the patient in which the calcification of the aortic valve did not progress among the patients, contained activated DPP-4 protein with approximately 4 times high level (FIG. 4c), the plasma of the patients with the severity 2 at about four times higher concentration, rather than the plasma of the patients with the severity 1 among the patients, contained a high concentration of DPP-4 protein (FIG. 4d), the plasma of the patient in which the calcification of the aortic valve progressed, rather than the plasma of the patient in which the calcification of the aortic valve did not progress among the patients, contained a high concentration of inactivated IGF-1 protein (FIG. 4e), and the plasma of the patients with the calcification severity, rather than the plasma of the patients without the calcification severity among the patients, contained a high concentration of inactivated IGF-1 protein (FIG. 4f).

The action mechanism of DPP-4 and IGF-1 involved in the onset of the aortic valve calcification disease provided by the present disclosure is shown as in FIG. 1. FIG. 1 is a schematic diagram showing an action mechanism of DPP-4 and IGF-1 involved in the onset of the aortic valve calcification disease. As shown in FIG. 1, in normal subjects, the IGF-1 binds to a receptor thereof (IGF-1R) located on the cell surface to perform signaling, thereby activating PI3K, ERK, AKT, etc., to prevent calcification of the valve. However, in the subject in which the aortic valve calcification disease occurs, the IGF-1 cleaved by DPP-4 to produce inactivated IGF-1, and the inactivated IGF-1 is unable to bind to IGF-1R located on the cell surface, which prevents activation of PI3K, ERK, AKT, etc., and thus, the calcification of the valve is induced, and the calcification level of the valve is also increased as the level of inactivated IGF-1 is increased.

Therefore, it may be appreciated that the DPP-4 and inactivated IGF-1 level may be used as biomarkers for diagnosing disease severity or calcification level in patients with the aortic valve calcification disease.

As another exemplary embodiment for achieving the purpose, the present disclosure provides a kit for diagnosing an aortic valve calcification disease comprising the composition for diagnosing an aortic valve calcification disease.

The kit of the present disclosure may be used to diagnose whether the aortic valve calcification disease occurs or the progression level thereof by measuring a protein level of the inactivated IGF-1 from isolated samples derived from a patient in which the aortic valve calcification disease is induced, and may include, but not particularly limited to, one or more other component compositions, solutions or devices suitable for analytical methods as well as antibodies for measuring the level of the protein.

Term "sample" of the present disclosure refers to a direct object which is isolated from a subject suspected of having the onset of the aortic valve calcification disease involving the valve calcification or a subject in which the onset of the aortic valve calcification disease is confirmed so as to measure an expression level of inactivated IGF-1 protein. For example, the sample may include, but not limited to, blood, serum, plasma, tissue samples, etc., that are isolated from mammals in which the aortic valve calcification disease is capable of being induced.

As a specific example, the kit of the present disclosure may be an ELISA kit for measuring a level of inactivated IGF-1 protein or a kit for analyzing a protein chip, wherein the kit may include, but not particularly limited to, a substrate, a suitable buffer solution, a secondary antibody labeled with a chromogenic enzyme or a fluorescent material, a chromogenic substance, etc., for immunological detection of the antibody. The substrate may be, but not particularly limited to, a 96-well plate synthesized with a nitrocellulose membrane or a polyvinyl resin, a 96-well plate synthesized with a polystyrene resin, a slide glass made of a glass, etc. The chromogenic enzyme may be, but not particularly limited to, peroxidase and alkaline phosphatase. The fluorescent material may be, but not particularly limited to, FITC, RITC, or the like. The color development substance liquid may be, but not particularly limited, ABTS (2,2'-azino-bis(3-ethylbenzothiazoline-6-sulfonic acid), OPD (o-phenylenediamine), or TMB (tetramethylbenzidine).

As another exemplary embodiment for achieving the purpose, the present disclosure provides a method for detecting inactivated IGF-1 to provide information necessary in the diagnosis of an aortic valve calcification disease, and a method for detecting inactivated IGF-1 to provide information necessary in the diagnosis of a progression level of the aortic valve calcification disease.

The method for detecting the inactivated IGF-1 provided by the present disclosure may be used as a means for diagnosing the calcification of the aortic valve or for diagnosing the progression level of calcification of the aortic valve that is already calcified according to the onset and progression of the aortic valve calcification disease, and thus, the method may be used as a method for diagnosing the aortic valve calcification disease or a method for diagnosing the progression level of the aortic valve calcification disease.

As an example, the method for detecting inactivated IGF-1 to provide information necessary for diagnosis of the aortic valve calcification disease provided by the present disclosure comprises: (a) measuring a level of inactivated insulin-like growth factor type 1 (IGF-1) protein from a biological sample isolated from a subject to be tested; and (b) comparing the measured protein level to a level measured from a biological sample isolated from a normal subject. Here, the biological sample of the subject may be, for example, blood, serum, plasma, tissue sample, etc., but is not particularly limited thereto, and the method may be performed to provide information for diagnosis of the aortic valve calcification disease.

In the above-described method, when the level of the inactivated IGF-1 protein measured from the biological sample of the subject to be tested is significantly increased as compared to the level measured from the biological sample isolated from the normal subject, it may be determined that the aortic valve calcification disease occurs in the subject to be tested, and when the level is not significantly increased, it may be determined that the aortic valve calcification disease is not induced in the subject to be tested. Herein, the method for measuring the level of the inactivated IGF-1 protein is the same as described above.

As another example, the method for detecting inactivated IGF-1 to provide information necessary for diagnosis of the progression level of the aortic valve calcification disease provided by the present disclosure comprises: (a) measuring a level of inactivated IGF-1 protein from a biological sample isolated from a subject to be tested; and (b) comparing the measured protein level to a level measured from a level measured from a standard sample with the severity of the aortic valve calcification disease. Here, the biological sample of the subject may be, for example, blood, serum, plasma, tissue sample, etc., but is not particularly limited thereto.

Further, the severity score may be a score of severity of aortic valve stenosis induced by the aortic valve calcification disease. As an example, the score may be a score measured by using a qualitative method using echo-brightness of the aortic valve observed by echocardiography in which the brightness is greater as the severity is higher as the calcification largely progresses, direct measurement of blood flow velocity flowing through the aortic valve on echocardiography by the Doppler technique, or a qualitative method of calculating a calcium score in a computed tomography scan. Reference values for these severity scores are already known in the art.

In this method, the standard sample with the severity of aortic valve calcification disease may be a sample showing the severity score of each aortic valve calcification disease. When the level of the inactivated IGF-1 protein measured from the biological sample of the subject to be tested is significantly increased as compared to the level measured from each standard sample, it may be determined that the aortic valve calcification disease occurred in the subject to be tested is more intensified than the severity of the aortic valve calcification disease shown in each standard sample, and when the level is not significantly increased, it may be determined that the aortic valve calcification disease occurred in the subject to be tested does not arrive at the severity of the aortic valve calcification disease. Here, the method for measuring the level of the inactivated IGF-1 protein is the same as described above, and the method may be performed to provide information for diagnosing the progression level of the aortic valve calcification disease.

### Example

Hereinafter, Examples of the present disclosure will be described in detail. However, those examples of the present disclosure will now be described, by way of example, and therefore, a scope of the present disclosure should not be construed as being restricted within the examples thereof.

### Example 1: Effects of IGF-1 and DPP-4 on osteoclastogenesis

### Example 1-1: Comparison in osteoclastogenesis level

Wild-type mice or eNOS-deficient mice which are animal models with the aortic valve calcification were treated with insulin-like growth factor type 1 (IGF-1) alone or treated with dipeptidyl-peptidase 4 (DPP-4) and IGF-1 at the same time. Then, ALP staining was performed on the aortic valve vascular smooth muscle tissue, thereby comparing the osteoclastogenesis level of tissue (FIG. 2a). Here, the aortic valve vascular smooth muscle tissue of the wild-type mice treated with nothing or the eNOS-deficient mice was used as a control group.

FIG. 2a are an image and a graph showing verification of the effect of DPP-4 on an action of IGF-1 inhibiting osteocytogenesis in the aortic valve vascular smooth muscle tissue of the wild-type mice or the eNOS-deficient mice. As shown in FIG. 2a, it was confirmed that the osteocytogenesis induced by the aortic valve vascular smooth muscle tissue of the wild-type or the eNOS-deficient mice tended to be alleviated by treatment with IGF-1, but the osteocytogenesis of the tissue was intensified by addition of DPP-4.

### Example 1-2: Comparison in activation of intracellular signaling protein

Further, in order to confirm whether the activation level of the protein involved in intracellular signaling was changed by osteocytogenesis according to DPP-4, the eNOS-deficient mice were treated with IGF-1 alone for 0, 5, 15 or 30 minutes, or treated with DPP-4 and IGF-1 at the same time, and then, the aortic valve vascular smooth muscle tissue was extracted from these mice. Then, a phosphorylation level of ERK or Akt which is a protein involved in intracellular signaling expressed in the extracted tissues was compared by western blot analysis (FIG. 2b). Here, β-actin was used as an internal control group.

FIG. 2b is a Western blot analysis image showing a change in the phosphorylation level of ERK or Akt which is the protein involved in intracellular signaling according to whether the DPP-4 is treated in the eNOS-deficient mice treated with IGF-1. As shown in FIG. 2b, it was confirmed that when the eNOS-deficient mice treated with IGF-1 was treated with DPP-4, the phosphorylation level of ERK or Akt which is the protein involved in intracellular signaling was reduced, and thus, it could be appreciated that the protein involved in the intracellular signaling was inactivated by DPP-4.

From the results shown in FIGS. 1a and 1b, it could be appreciated that the action of IGF-1 capable of alleviating the osteocytogenesis was inhibited by the treatment with DPP-4, and this inhibitory effect inactivated the protein involved in the intracellular signaling.

### Example 2: Analysis of correlation between aortic valve calcification and inactivated IGF-1

From the results of Example 1 above, the possibility that DPP-4 will act to inactivate IGF-1 emerged. That is, since DPP-4 is known to exhibit activity as a peptidase, it was expected that the DPP-4 cleaved two amino acids at the N-terminal of IGF-1 to form a inactivated form of IGF-1, thereby eliminating the osteocytogenesis alleviation effect of IGF-1.

First, each plasma was obtained from the wild-type mice or the eNOS-deficient mice, and the concentration of DPP-4 protein contained in each plasma was measured (FIG. 3a). Here, the concentration of DPP-4 protein was measured using an ELISA kit comprising immobilized anti-DPP-4 antibody, biotin-conjugated anti-DPP-4 antibody for detection, and HRP-conjugated streptavidin.

FIG. 3a is a graph showing comparison results in view of concentrations of DPP-4 protein contained in the plasma of wild-type mice or eNOS-deficient mice. As shown in FIG. 3a, it was confirmed that DPP-4 protein was contained at a high concentration in the plasma of eNOS-deficient mice, rather than the plasma of wild-type mice.

Next, the protein concentration of inactivated IGF-1 contained in the plasma of wild-type or eNOS-deficient mice that were treated or not treated with sitagliptin known as an inhibitor of DPP-4 was measured, taking advantage of the fact that the inactivated form of IGF-1 specifically binds to IGFBP3 which is one type of IGF binding protein.

Specifically, the level of inactivated IGF-1 contained in the sample was measured by measuring a level of total IGF-1 contained in each plasma sample, reacting the sample with an IGFBP3-coated ELISA kit, followed by washing to select only the inactivated IGF-1 among the total IGF-1 contained in the sample, and adding a labeled anti-IGF-1 antibody to measure the level of inactivated IGF-1 (FIG. 3b).

FIG. 3b is a graph showing comparison results of protein concentrations of inactivated IGF-1 bound to IGFBP3 contained in the plasma of wild-type mice or eNOS-deficient mice treated or not treated with sitagliptin. As shown in FIG. 3b, when treated with sitagliptin which is an inhibitor of DPP-4, it was confirmed that the protein concentration of inactivated IGF-1 bound to IGFBP3 was reduced.

From the above results, it could be appreciated that the level of inactivated IGF-1 in the plasma directly reflected the activity of DPP-4, and thus, it was analyzed that the inactivated IGF-1 was usable as a biomarker for predicting the aortic valve calcification.

### Example 3: Verification using patients with aortic valve calcification disease

### Example 3-1: DPP-4 protein level in plasma of patient with aortic valve calcification disease

The concentration of DPP-4 protein was measured from the plasma of normal people or the plasma of the patient with the aortic valve calcification disease (FIG. 4a).

FIG. 4a is a graph showing comparison results of concentrations of DPP-4 protein contained in the plasma of normal people or the patient in which the aortic valve calcification disease was induced. As shown in FIG. 4a, it was confirmed that DPP-4 protein was contained at a high concentration in the plasma of the patient in which the aortic valve calcification disease was induced, rather than the plasma of normal people.

### Example 3-2: Analysis of correlation between calcification and DPP-4 protein level

The concentration of DPP-4 protein was measured from the plasma of the patient in which the calcification of the aortic valve did not progress or the patient in which the calcification of the aortic valve progressed, among patients in which the aortic valve calcification disease was induced (FIG. 4b).

FIG. 4b is a graph showing comparison results of concentrations of DPP-4 protein contained in the plasma of the patient in which the calcification of the aortic valve did not progress or the patient in which the calcification of the aortic valve progressed, among the patients in which the aortic valve calcification disease was induced. As shown in FIG. 4b, it was confirmed that DPP-4 protein was contained at a high concentration in the plasma of the patient in which the calcification of the aortic valve progressed rather than the patient in which the calcification of the aortic valve did not progress, among the patients in which the aortic valve calcification disease was induced.

### Example 3-3: Analysis of correlation between calcification level and activated DPP-4 protein level

The concentration of activated DPP-4 protein was measured by measuring the enzymatic activity of DPP-4 from the plasma of the patient in which the calcification of the aortic valve did not progress or the patient in which the calcification of the aortic valve progressed, among patients in which the aortic valve calcification disease was induced.

That is, when the plasma sample is added to a substrate (Gly-Pro-AMC) capable of being cleaved by DPP-4, the substrate is degraded by DPP-4 contained in the plasma sample, and AMC(7-Amino-4-Methyl Coumarin) is separated, and fluorescence (Ex 360 nm, Em 460 nm) is emitted by the separated AMC. According to the above-described principle, the plasma sample and the substrate were reacted, the fluorescence value emitted therefrom was measured to calculate the activity of DPP-4, and the calculated activity was converted to measure the concentration of activated DPP-4 protein contained in the plasma sample (FIG. 4c) .

FIG. 4c is a graph showing comparison results of concentrations of activated DPP-4 protein contained in the plasma of the patient in which the calcification of the aortic valve did not progress or the patient in which the calcification of the aortic valve progressed, among the patients in which the aortic valve calcification disease was induced. As shown in FIG. 4c, it was confirmed that the plasma of the patient in which the calcification of the aortic valve progressed, rather than the plasma of the patient in which the calcification of the aortic valve did not progress, among the patients in which the aortic valve calcification disease was induced, contained about four times high level of concentration of activated DPP-4 protein.

### Example 3-4: Analysis of correlation between calcification degree and DPP-4 protein level

The concentration of the DPP-4 protein was measured from the plasma of the patient in which the aortic valve calcification disease with severity 1 or 2 was induced (FIG. 4d) .

FIG. 4d is a graph showing comparison results of concentrations of DPP-4 protein contained in the plasma of the patient in which the aortic valve calcification disease with severity 1 or 2 was induced. As shown in FIG. 4d, it was confirmed that the DPP-4 protein was contained at a high concentration in the plasma of the patient with the severity 2 rather than the patient with the severity 1 among the patients in which the aortic valve calcification disease was induced.

### Example 3-5: Analysis of correlation between calcification and inactivated IGF-1 protein level

The concentration of the inactivated IGF-1 protein was measured from the plasma of the patient in which the calcification of the aortic valve did not progress or the patient in which the calcification of the aortic valve progressed among patients in which the aortic valve calcification valve was induced (FIG. 4e).

FIG. 4e is a graph showing comparison results of concentrations of inactivated IGF-1 protein contained in the plasma of the patient in which the calcification of the aortic valve did not progress or the patient in which the calcification of the aortic valve progressed among the patients in which the aortic valve calcification disease was induced. As shown in FIG. 4e, it was confirmed that the inactivated IGF-1 protein was contained at a high concentration in the plasma of the patient in which the calcification of the aortic valve progressed, rather than the patient in which the calcification of the aortic valve calcification did not progress among the patients in which the aortic valve calcification disease was induced.

### Example 3-6: Analysis of correlation between calcification level and inactivated IGF-1 protein level

The concentration of the inactivated IGF-1 protein was measured from the plasma of the patient in which the aortic valve calcification disease without the calcification severity was induced or the patient in which the aortic valve calcification disease with the calcification severity was induced, among the patients in which the aortic valve calcification disease with the progress of calcification was induced (FIG. 4f).

FIG. 4f is a graph showing comparison results of concentrations of inactivated IGF-1 protein contained in the plasma of the patient in which the aortic valve calcification disease without the calcification severity is induced or the patient in which the aortic valve calcification disease with the calcification severity is induced, among the patients in which the aortic valve calcification disease with the progress of calcification is induced. As shown in FIG. 4f, it was confirmed that the inactivated IGF-1 protein was contained at a high concentration in the plasma of the patient with the calcification severity, rather than the patient without the calcification severity among the patients in which the aortic valve calcification disease was induced.

From the results of FIGS. 4a to 4f, it was found that the level of the DPP4 or the inactivated IGF-1 was increased in patients in which the aortic valve calcification disease was induced rather than in normal people, increased in the patient in which the calcification of the aortic valve processed rather than the patient in which the aortic valve of the calcification did not progress among the patients in which the aortic valve calcification disease was induced, and further increased in the patient with high calcification severity rather than the patient with low calcification severity even among the patients in which the aortic valve calcification disease with the progress of calcification was induced.

Therefore, it could be appreciated that the DPP-4 and inactivated IGF-1 level may be used as biomarkers for diagnosing disease severity or calcification level in patients with the aortic valve calcification disease.

### [Industrial Applicability]

According to the present disclosure, when using the composition or the kit for diagnosing an aortic valve calcification disease, it is possible to determine whether the aortic valve calcification disease involving the calcification of aortic valves has occurred, and to determine the progression level of the aortic valve calcification disease which has already occurred, and thus the composition or the kit of the present disclosure may be used for more effectively diagnosing and treating the aortic valve calcification disease.

The following detailed description of the present disclosure that can be fully recognized and deduced by those skilled in the art in the technical field of the present disclosure may be omitted, and various modifications can be made within a range in which the technical idea or the essential configuration of the present disclosure is not changed in addition to the specific examples described in the present specification. Therefore, the present disclosure may be practiced in different ways from the specifically described and exemplified contents in the present specification, which can be understood by those skilled in the art.

## Claims

1. A composition for diagnosing an aortic valve calcification disease comprising an agent for measuring a protein level of inactivated insulin-like growth factor type 1 (IGF-1), in which 2 to 10 amino acids at the N-terminal of IGF-1 are cleaved.

2. The composition according to claim 1, wherein the inactivated IGF-1 comprise an amino acid sequence of SEQ ID NO: 2.

3. The composition according to claim 1, wherein the agent for measuring a protein level comprises an antibody or an aptamer specifically binding to the inactivated IGF-1.

4. A kit for diagnosing an aortic valve calcification disease comprising the composition according to any one of claims 1 to 3.

5. The kit according to claim 4, wherein the kit is protein chip kit.

6. A method for detecting inactivated IGF-1 to provide information necessary for diagnosis of the aortic valve calcification disease comprises:
(a) measuring a level of inactivated insulin-like growth factor type 1 (IGF-1) protein from a biological sample isolated from a subject to be tested; and
(b) comparing the measured protein level to a level measured from a biological sample isolated from a normal subject.

7. The method according to claim 6, wherein the sample is blood, serum, plasma, or tissue sample.

8. A method for detecting inactivated IGF-1 to provide information necessary for diagnosis of the progression level of the aortic valve calcification disease comprises:
(a) measuring a level of inactivated IGF-1 protein from a biological sample isolated from a subject to be tested; and
(b) comparing the measured protein level to a level measured from a level measured from a standard sample with the severity of the aortic valve calcification disease.

9. The method according to claim 8, wherein the sample is blood, serum, plasma, or tissue sample.

10. The method according to claim 8, wherein the standard sample with the severity of the aortic valve calcification disease is a sample with a score of severity of the aortic valve calcification disease.
